# EUROPEAN PATENT APPLICATION

(11) **EP 2 361 615 A1**
(43) Date of publication of application: **31.08.2011**
(21) Application number: 10003718.3
(22) Date of filing: 07.04.2010
(51) Int. Cl.: A61K 9/22, A61K 31/519

(54) **Dipyridamole prolonged-release tablet**

(30) Priority: 19.02.2010 IN DE03792010
(71) Applicant: Alfred E. Tiefenbacher GmbH & Co. KG, 22767 Hamburg (DE)
(72) Inventor: Pasahn, Manohar, Thane (West) 400 602 Maharashtra (IN); Chary, Rallabandi Bala Ramesha, Kukatpall Hyderabad - 500 072 (IN); Jagadeesh, Thipperudraiah, Hiriyur, Chitradurga (Dist.) Karnataka (IN); Joshi, Abhay Ramakant, Kolhapur (Dist) Maharashtra (IN)
(74) Representative: von Seebach, Malte

(57) **Abstract**

The present invention relates to a gastro-retentive, prolonged-release tablet formulation comprising in the intragranular phase a pharmaceutically active ingredient and a water-swellable polymer, and in the extragranular phase hydroxypropyl methyl cellulose (HPMC). The tablet of the present invention is further characterized in that it contains a gas generating agent, thereby causing the tablet to float on the gastric juice. The tablet is particularly suitable for dipyridamole which is a drug that inhibits thrombus formation. The present invention thus provides an alternative to the commercially available dipyridamole containing pharmaceutical compositions marketed under the tradenames Aggrenox^{®} and Persantine^{®}.

## Description

The present invention relates to a gastro-retentive, prolonged-release tablet formulation, i.e. intragastric floating tablet, and to a process for manufacturing said tablet.

Gastro-retentive dosage forms are known for releasing a drug into a portion of the gastrointestinal tract defined by the stomach, duodenum and proximal ileum. It is generally known that the retention time of an orally administered controlled drug delivery system in the gastrointestinal tract as well as the rate at which the controlled drug delivery system moves from the stomach to the colon are factors to be considered in the design of the oral controlled delivery system. It is thus known that a prolonged period of retention of the drug delivery system in the stomach, as achieved by gastro-retentive dosage forms, is beneficial for various types of drugs, e.g. for a drug that is most effectively absorbed in the stomach, duodenum or proximal ileum. Therefore, gastro-retentive dosage forms are particularly useful for drugs exhibiting a narrow absorption window in the upper gastrointestinal tract, such as drugs poorly soluble in a neutral or basic environment as encountered in the intestine. Gastro-retentive dosage forms are therefore particularly suitable for drugs which are soluble in a gastric juice, i.e. in an acidic environment, as basic or weak basic drugs, and which may precipitate and/or dissolve incompletely in the higher intestinal pH-environment. Precipitation or incomplete dissolution of the drug in the intestine usually causes a decrease of drug absorption and thus a low bioavailability.

In order to improve the bioavailability of such pH-dependent drugs gastro-retentive dosage forms have been suggested which provide for a prolonged-release of the drug into the stomach. One known approach for achieving gastric retention involves a composition containing highly swellable polymers in admixture with a gas generating agent. The composition swells in the stomach and the gas generated is entrapped therein, whereby the composition's density is decreased, which causes buoyancy.

The European patent EP 0 976 395 discloses a tablet for prolonged-release of a drug into the stomach. The tablet is prepared using a wet granulation technique, wherein a mixture of a drug, such as metformin, and hydroxypropyl methyl cellulose (HPMC or hypromellose) is granulated. The granules are subsequently layered with a coating containing a source of carbon dioxide. Thereafter, the coated granules are blended with tabletting aids and an organic acid, and finally compressed into tablets. The organic acid contained in the tablet should initiate and promote the release of carbon dioxide.

WO 2007/048223 describes a gastro-retentive, prolonged-release dosage form which is characterized in that the drug is contained within a hydrophilic swellable matrix consisting of a combination of hydroxypropyl cellulose (HPC) and HPMC in a weight reassure of HPC to HPMC ranging from 80:20 to 20:80. The hydrophilic swellable matrix further contains a gas generating agent such as an alkali metal carbonate, an alkaline earth metal carbonate or an alkali metal hydrogen carbonate.

The European patent EP 1 138 320 discloses a gastro-retentive dosage form which floats on the gastric juice without requiring the presence of a gas generating agent. Instead, a particular co-processed mixture of polyvinylacetate and polyvinylpyrrolidone is used as a matrix forming component. Such a co-processed mixture is commercially available under the tradename Kollidon^{®} SR. The polyvinylacetate/polyvinylpyrrolidone component does not form a water swellable matrix, but a hydrophobic matrix. Due to the high dry-binding capacity of the co-processed material very porous floating matrix tablets can be prepared, which is achieved by compressing the tablet mass into the tablets using unusually low pressures of less than 100 MPa.

The European patent EP 1 745 775 discloses a gastro-retentive dosage form, wherein a drug is granulated with a mixture of a weak gelling agent, a strong gelling agent and a gas generating agent. The weak gelling agent is a co-processed material of microcrystalline cellulose and sodium carboxymethyl cellulose (CMC-Na), whereas the strong gelling agent is preferably selected from methyl cellulose (MC), HPMC, HPC (i.e. not low-substituted HPC), CMC-Na, xanthan gum, and the like.

A monolithic floating matrix tablet containing dipyridamole is described in Indian J Pharm. Sci. 2007, 69(2), 219-225. The intragastric floating tablet contains dipyridamole, which is a poorly soluble weak base dissolving rapidly in the stomach, but incompletely in the intestine, and polyethylene oxide (PEO) as a water-swellable matrix forming polymer. The tablet further contains sodium bicarbonate as a gas generating agent and lactose and/or pregelatinized starch. The monolithic floating matrix tablet is prepared by direct compression.

Dipyridamole is used in conjunction with acetylsalicylic acid in the secondary prevention of stroke and transient ischaemic attack. Said combination is commercially available under the tradenames Aggrenox^{®} and Persantine^{®} As dipyridamole exhibits a pH-dependent absorption profile the bioavailability is decreased due to the narrow absorption window. In order to improve the bioavailability of dipyridamole European patent EP 0 068 191 suggests to add acidic excipients, preferably organic acids such as tartaric acid, citric acid, fumaric acid, succinic acid, malic acid, ascorbic acid, adipic acid and the like to dipyridamole containing pharmaceutical compositions for oral administration. The acidic microenvironment established in the pharmaceutical composition facilitates the absorption of dipyridamole even from the neutral to alkaline environment of the intestine.

A dipyridamole containing delayed release formulation is disclosed in the European patent EP 0 032 562. The formulation is characterized in that spheroidal particles containing dipyridamole and an acid are coated with an acid-insoluble lacquer that is, however, soluble in the intestinal juice.

The European patent EP 0 257 344 discloses a pharmaceutical combination comprising dipyridamole containing delayed release pellets and acetylsalicylic acid containing tablets, wherein both formulations are packed into a capsule.

Various formulations have been suggested in the state of the art for providing gastro-retentive, prolonged-release properties to a tablet. However, it appears that no generally applicable concept has been provided so far, which allows the prolonged-release profile of a tablet to be specifically adjusted to the respective gastro-retentive properties. For example, a prolonged-release profile according to which a drug is released over a prolonged period of time, such as twelve hours, is disadvantageous if the tablet floats on the gastric juice for only six hours, since a drug absorbed only in the stomach and/or proximal intestine would be released in the lower part of the intestine where the drug cannot be absorbed due to its low solubility in a neutral of alkaline environment. In such a case, it would be advantageous to adapt the release profile to the total floating time of the tablet, so that the drug is essentially entirely released from the tablet within the total floating time.

Therefore, it was an object of the present invention to provide a gastro-retentive, prolonged-release tablet, i.e. an intragastric floating tablet, in which the prolonged-release profile can be specifically adjusted to the respective floating properties. This object is solved by the subject matter as defined in the claims.

The present invention thus relates to an intragastric floating tablet, comprising
a) in the intragranular phase: a pharmaceutically active ingredient and a water-swellable polymer, and
b) in the extragranular phase: hydroxypropyl methyl cellulose (HPMC), wherein the tablet contains a gas generating agent.

The intragastric floating tablet of the present invention can be prepared using a dry granulation or wet granulation technique, wherein the latter is preferred. In a preferred embodiment of the present invention the tablet contains the gas generating agent exclusively in the extragranular phase. Examples of the gas generating agent include alkali metal or alkaline earth metal carbonates, e.g. sodium or calcium carbonate, alkali metal hydrogen carbonates, e.g. sodium bicarbonate, and alkali metal sulfites, e.g. sodium metabisulfite.

Preferably, the tablet of the present invention does not contain an acid as an excipient capable to initiate and promote the gas generation from the gas generating agent.

In a preferred embodiment of the present invention the tablet further contains as an intragranular component one or more fillers such as microcrystalline cellulose, calcium hydrogen phosphate, lactose, mannitol, sorbitol and the like, wherein microcrystalline cellulose, calcium hydrogen phosphate and lactose are more preferred. It has been found that the intragranular addition of a filler allows a fine tuning of the floating properties, whereas the prolonged-release profile of the tablet is affected by the filler to a much lesser extent. Usually, the total floating time of the tablet decreases the higher the amount of the filler in the intragranular phase. In a preferred embodiment of the present invention a filler, if present, is only contained in the intragranular phase. It has been found that the mass ratio of the water-swellable polymer to the one or more fillers in the intragranular phase should be in the range of 10:1 to 0.5:1, preferably 5:1 to 1:1, and more preferred 2:1 to 1:1.

The tablet of the present invention contains a water-swellable polymer in the intragranular phase. Examples of the water-swellable polymer include HPMC, polyethylene oxide (PEO), hydroxypropyl cellulose (HPC), methyl cellulose (MC), sodium carboxymethyl cellulose (CMC-Na), pregelatinized starch, tragacanth, pectin, polyacrylic acid, gelatine, xanthan gum, cross-linked polyvinylpyrrolidone (crospovidone), and mixtures thereof.

The tablet of the present invention is in particular suitable for high drug loadings. Hence, the tablet preferably contains the pharmaceutically active ingredient in an amount of at least 40% by weight, preferably at least 50% by weight, and more preferred at least 55% by weight, based on the weight of the components constituting the intragranular phase. In order to safeguard both the prolonged-release and floating properties the drug amount in the intragranular phase should not exceed 90% by weight, preferably 80% by weight, based on the weight of the components constituting the intragranular phase.

Generally, the pharmaceutically active ingredient is only contained in the intragranular phase. In a preferred embodiment of the present invention, the tablet does not contain in the extragranular phase components other than HPMC, the gas generating agent, and optionally a lubricant such as magnesium stearate, sodium stearyl fumarate, zinc stearate, stearic acid, glyceryl behenate, glyceryl monostearate, and the like.

The tablet according to the present invention preferably contains HPMC as the water-swellable polymer, i.e. HPMC is contained both in the intragranular phase and the extragranular phase.

The tablet of the present invention allows the prolonged-release profile to be specifically adjusted to the respective floating properties. In this regard, the floating properties of the tablet are essentially controlled by the amount of the HPMC contained in the extragranular phase.

In a preferred embodiment of the present invention the total floating time of the tablet is from 5 h to 8 h, preferably from 5 h 30 min to 7 h 30 min, and more preferred form 6 h to 7 h, measured in 200 ml 0.1 N hydrochloric acid (pH 1.2) with gentle stirring (100 rpm) using a magnetic stirrer at 37 ± 0.5 °C. The floating properties of the tablet were studied by placing the tablet from top in 200 ml of 0.1 N hydrochloric acid in a transparent beaker, and checking the time taken for floating (floating lag time) and duration of floating (total floating time).

The prolonged-release profile of the tablet is adjusted, so that 90 to 100 % of the amount of the pharmaceutically active ingredient is released from the tablet after 5 h to 8 h, preferably 5 h 30 min to 7 h 30 min, and more preferred 6 h to 7 h, measured in 900 ml 0.1 N hydrochloric acid (pH 1.2) at 37 ± 0.5 °C (basket apparatus, 100 rpm). The release profile of the tablet of the present invention is measured according to the "dissolution test for solid dosage forms" disclosed in chapter 2.9.3 of the European Pharmacopeia 6.6.

Preferably, the prolonged-release profile is adjusted so that 90 to 100 % of the amount of the drug is released from the tablet during the total floating time ± 30 min. The floating lag time observed in the tablet of the present invention is usually less than 2 min, preferably less than 30 sec and more preferred less than 20 sec.

The present invention further relates to a process for preparing the intragastric floating tablet as described above, comprising the steps of:
i) preparing granules containing a pharmaceutically active ingredient, a water-swellable polymer and optionally a gas generating agent,
ii) providing a tablet mass containing the granules according to step (i), a gas generating agent and HPMC, and
iii) compressing the tablet mass.

Preferably the granules are prepared by wet granulation, more preferably by a wet granulation which is conducted with an aqueous granulation liquid. A suitable granulation liquid includes a mixture of water and an alcohol, preferably a mixture of water and isopropyl alcohol.

The gastro-retentive, prolonged-release tablet of the present invention is useful for drugs that are primarily absorbed in the stomach, duodenum and/or proximal ileum. These drugs are characterized in that they exhibit a high rate of solubilisation in the gastric (acidic) juice, which is dramatically reduced in the intestinal (alkaline) juice. Therefore, the intragastric floating tablet of the present invention is in particular suited for pH-dependent drugs, such as weakly basic drugs, having a solubility difference between pH 1.2 and pH 6.8 ≥ 10-fold, preferably ≥40-fold, more preferred ≥ 100-fold. Examples of the drug suited for the tablet of the present invention include captopril, carvedilol, chlordiazepoxide, cinnarizine, diltiazem, dipyridamole, diazepam, enoxacin, erlotinib, glimepiride, indapamide, ketanserine, ketoconazole, lamotrigine, leflunomide, metformin, mopidamole, ondansetron, papaverine, propiverine, propranolol, quetiapine, repaglinide, sibutramine, ursodeoxycholic acid, vardenafil, verapamile and ziprasidone, and pharmaceutically acceptable salts thereof. The tablet of the present invention is particularly suitable for dipyridamole, erlotinib, leflunomide, metformin, quetiapine, repaglinide, sibutramine, vardenafil and ziprasidone, and pharmaceutically acceptable salts thereof.

The tablet of the present invention may contain dipyridamole as the pharmaceutically acceptable ingredient. In this regard, an alternative to the dipyridamole/acetylsalicylic acid combination, which is commercially available under the tradenames Aggrenox^{®} and Persantine^{®}, is provided, wherein the pharmaceutical combination is a unit dosage form comprising (a) the tablet according to the present invention, wherein the pharmaceutical active ingredient is dipyridamole or a pharmaceutically acceptable salt thereof, and (b) an oral dosage form containing acetylsalicylic acid or a pharmaceutically acceptable salt thereof. Preferably, the unit dosage form is a capsule for oral administration.

The present invention is further illustrated by the following examples. The floating lag time and the total floating time were determent by placing the tablet from top in 200 ml of 0.1 N hydrochloric acid in a transparent beaker with gentle stirring (100 rpm) using a magnetic stirrer at 37 ± 0.5 °C, and checking the time taken for floating (floating lag time) and duration of floating (total floating time). The dissolution profile was determined according to the dissolution test for solid dosage forms described in chapter 2.9.3 of the European Pharmacopeia 6.6 using 900 ml 0.1 N hydrochloric acid (pH 1.2) at 37 ± 0.5 °C (basket apparatus, 100 rpm).

### Example 1

| Ingredient | Milligram / tablet |
|---|---|
| **Stage-A (Dry mix)** | |
| Dipyridamole | 200.00 |
| Hypromellose (Methocel^{®} K100 LV Premium) | 80.00 |
| Cellulose, Microcrystalline (Avicel^{®} PH 101) | 62.00 |
| **Stage-B (Binder Solution)** | |
| Water, Purified / Isopropyl Alcohol (30:70) | q.s. |
| **Stage-C (Blending and lubrication)** | |
| *Dipyridamole granules* | *342.00* |
| Hypromellose (Methocel^{®}K100 LV Premium) | 20.00 |
| Sodium Bicarbonate | 10.00 |
| Magnesium Stearate | 8.00 |
| *Total tablet weight* | *380. 00* |

### Process:

A dry mix containing dipyridamole, hypromellose (HPMC) and microcrystalline cellulose was treated with a binder solution containing water and isopropyl alcohol. The wet mass was then milled to granules which have been subsequently dried. Thereafter, the granules were mixed with HPMC, sodium bicarbonate and magnesium stearate to obtain a tablet mass which was compressed into the tablet.

### Dissolution test (0. N Hydrochloric Acid, 900 ml, basket-100 rpm):

| Time [hour] | % Drug Dissolved |
|---|---|
| 1 | 23 |
| 2 | 49 |
| 3 | 68 |
| 6 | 95 |
| 8 | 98 |

### Floating properties (in 0.1 N Hydrochloric Acid):

| | |
|---|---|
| Floating lag time: | 15 sec |
| Total floating time: | 6 h |

### Example 2

| Ingredient | Milligram / tablet |
|---|---|
| **Stage-A (Dry mix)** | |
| Dipyridamole | 200.00 |
| Hypromellose (Methocel^{®}K100 LV) | 80.00 |
| Lactose Monohydrate | 62.00 |
| **Stage-B (Binder Solution)** | |
| Water, Purified / Isopropyl Alcohol (30:70) | q.s. |
| **Stage-C (Blending and lubrication)** | |
| *Dipyridamole granules* | *342.00* |
| Hypromellose (Methocel^{®} K100 LV) | 20.00 |
| Sodium Bicarbonate | 10.00 |
| Magnesium Stearate | 8.00 |
| *Total tablet weight* | *380. 00* |

### Process:

The tablet was prepared as described in Example 1.

### Dissolution test (0.1N Hydrochloric Acid, 900 ml, basket-100 rpm):

| Time [hour] | % Drug Dissolved |
|---|---|
| 1 | 35 |
| 2 | 60 |
| 3 | 83 |
| 6 | 97 |
| 8 | 97 |

### Floating properties (in 0.1 N Hydrochloric Acid):

| | |
|---|---|
| Floating lag time: | 15 sec |
| Total floating time: | 5 h 15 min |

### Example 3

| Ingredient | Milligram / tablet |
|---|---|
| **Stage-A (Dry mix)** | |
| Dipyridamole | 200.00 |
| Dicalcium Phosphate | 62.00 |
| Hypromellose (Methocel^{®} K100 LV) | 80.00 |
| **Stage-B (Binder Solution)** | |
| Water, Purified / Isopropyl Alcohol (30:70) | q.s. |
| **Stage-C (Blending and lubrication)** | |
| *Dipyridamole granules* | *342.00* |
| Hypromellose (Methocel^{®} K100 LV) | 20.00 |
| Sodium Bicarbonate | 10.00 |
| Magnesium Stearate | 8.00 |
| *Total tablet weight* | *380.00* |

### Process:

The tablet was prepared as described in Example 1.

### Dissolution test (0.1 N Hydrochloric Acid, 900 ml, basket-100 rpm):

| Time [hour] | % Drug Dissolved |
|---|---|
| 1 | 33 |
| 2 | 58 |
| 3 | 79 |
| 6 | 101 |
| 8 | 101 |

### Floating properties (in 0.1 N Hydrochloric Acid):

| | |
|---|---|
| Floating lag time: | 20 sec |
| Total floating time: | 5 h 30 min |

### Example 4

| Ingredient | Milligram / tablet |
|---|---|
| **Stage-A (Dry mix)** | |
| Dipyridamole | 200.00 |
| Hypromellose (Methocel^{®} K100 LV) | 80.00 |
| **Stage-B (Binder Solution)** | |
| Water, Purified / Isopropyl Alcohol (30:70) | q.s. |
| **Stage-C (Blending and lubrication)** | |
| *Dipyridamole granules* | *280.00* |
| Hypromellose (Methocel^{®} K100 LV) | 20.00 |
| Sodium Bicarbonate | 10.00 |
| Magnesium Stearate | 8.00 |
| *Total tablet weight* | *318.00* |

### Process:

The tablet was prepared as described in Example 1.

### Dissolution test (0. N Hydrochloric Acid, 900 ml, basket-100 rpm):

| Time [hour] | % Drug Dissolved |
|---|---|
| 1 | 30 |
| 2 | 52 |
| 3 | 71 |
| 6 | 99 |
| 8 | 101 |

### Floating properties (in 0.1 N Hydrochloric Acid):

| | |
|---|---|
| Floating lag time: | 15 sec |
| Total floating time: | 6 h 15 min |

### Example 5

| Ingredient | Milligram / tablet |
|---|---|
| **Stage-A (Dry mix)** | |
| Dipyridamole | 200.00 |
| Cellulose, Microcrystalline | 62.00 |
| Sodium Bicarbonate | 10.00 |
| Hypromellose (Methocel^{®} K100 LV) | 100.00 |
| **Stage-B (Binder Solution)** | |
| Water, Purified / Isopropyl Alcohol (30:70) | q.s. |
| **Stage-C (Blending and lubrication)** | |
| *Dipyridamole granules* | *372.00* |
| Magnesium Stearate | 8.00 |
| *Total tablet weight* | *380.00* |

### Process:

The tablet was prepared as described in Example 1.

### Dissolution test (0.1N Hydrochloric Acid, 900 ml, basket-100 rpm):

| Time [hour] | % Drug Dissolved |
|---|---|
| 1 | 32 |
| 2 | 52 |
| 3 | 69 |
| 6 | 99 |
| 8 | 100 |

### Floating properties (in 0.1 N Hydrochloric Acid):

| | |
|---|---|
| Floating lag time: | 15 sec |
| Total floating time: | 6 h |

### Example 6

| Ingredient | Milligram / tablet |
|---|---|
| **Stage-A (Dry mix)** | |
| Dipyridamole | 200.00 |
| Hypromellose (Methocel^{®}K100 LV) | 142.00 |
| **Stage-B (Binder Solution)** | |
| Water, Purified / Isopropyl Alcohol (30:70) | q.s. |
| **Stage-C (Blending and lubrication)** | |
| *Dipyridamole granules* | *342. 00* |
| Hypromellose (Methocel^{®} K100 LV) | 20.00 |
| Sodium Bicarbonate | 10.00 |
| Magnesium Stearate | 8.00 |
| *Total tablet weight* | *380. 00* |

### Process:

The tablet was prepared as described in Example 1.

### Dissolution test (0.1 N Hydrochloric Acid, 900 ml, basket-100 rpm):

| Time [hour] | % Drug Dissolved |
|---|---|
| 1 | 22 |
| 2 | 37 |
| 3 | 53 |
| 6 | 84 |
| 8 | 97 |

### Floating properties (in 0.1 N Hydrochloric Acid):

| | |
|---|---|
| Floating lag time: | 15 sec |
| Total floating time: | 7 h |

### Example 7

| Ingredient | Milligram / tablet |
|---|---|
| **Stage-A (Dry mix)** | |
| Dipyridamole | 200.00 |
| Hypromellose (Methocel^{®}K100 LV) | 100.00 |
| Cellulose, Microcrystalline | 42.00 |
| **Stage-B (Binder Solution)** | |
| Water, Purified / Isopropyl Alcohol (30:70) | q.s. |
| **Stage-C (Blending and lubrication)** | |
| *Dipyridamole granules* | *3-12.00* |
| Hypromellose (Methocel^{®} K100 LV) | 20.00 |
| Sodium Bicarbonate | 10.00 |
| Magnesium Stearate | 8.00 |
| *Total tablet weight* | *380.00* |

### Process:

The tablet was prepared as described in Example 1.

### Dissolution test (0.1 N Hydrochloric Acid, 900 ml, basket-100 rpm):

| Time [hour] | % Drug Dissolved |
|---|---|
| 1 | 28 |
| 2 | 47 |
| 3 | 66 |
| 6 | 97 |
| 8 | 100 |

### Floating properties (in 0. 1N Hydrochloric Acid):

| | |
|---|---|
| Floating lag time: | 10 sec |
| Total floating time: | 8 h |

### Example 8

| Ingredient | Milligram / tablet |
|---|---|
| **Stage-A (Dry mix)** | |
| Dipyridamole | 200.00 |
| Hypromellose (Methocel^{®} K100 LV) | 60.00 |
| Cellulose, Microcrystalline | 82.00 |
| **Stage-B (Binder Solution)** | |
| Water, Purified / Isopropyl Alcohol (30:70) | q.s. |
| **Stage-C (Blending and lubrication)** | |
| *Dipyridamole granules* | *342.00* |
| Hypromellose (Methocel^{®} K100 LV) | 20.00 |
| Sodium Bicarbonate | 10.00 |
| Magnesium Stearate | 8.00 |
| *Total tablet weight* | *380.00* |

### Process:

The tablet was prepared as described in Example 1.

### Dissolution test (0.1 N Hydrochloric Acid, 900 ml, basket-100 rpm):

| Time [hour] | % Drug Dissolved |
|---|---|
| 1 | 38 |
| 2 | 61 |
| 3 | 86 |
| 6 | 102 |
| 8 | 102 |

### Floating properties (in 0.1 N Hydrochloric Acid):

| | |
|---|---|
| Floating lag time: | 10 sec |
| Total floating time: | 5 h 30 min |

### Comparative Example 1: HPMC is replaced with polyethylene oxide (Polyox™ WSR 303 NF)

| Ingredient | Milligram / tablet |
|---|---|
| **Stage-A (Dry mix)** | |
| Dipyridamole | 200.00 |
| Cellulose, Microcrystalline | 62.00 |
| **Stage-B (Binder Solution)** | |
| Water, Purified / Isopropyl Alcohol (30:70) | q.s. |
| **Stage-C (Blending and lubrication)** | |
| *Dipyridamole granules* | *262.00* |
| Polyethylene Oxide (Polyox™ WSR 303 NF) | 100.00 |
| Sodium Bicarbonate | 10.00 |
| Magnesium Stearate | 8.00 |
| *Total tablet weight* | *380.00* |

### Process:

The tablet was prepared as described in Example 1.

### Dissolution test (0.1 N Hydrochloric Acid, 900 ml, basket-100 rpm):

| Time [hour] | % Drug Dissolved |
|---|---|
| 1 | 18 |
| 2 | 30 |
| 3 | 39 |
| 6 | 63 |
| 8 | 74 |

### Floating properties (in 0. 1N Hydrochloric Acid):

| | |
|---|---|
| Floating lag time: | 15 sec |
| Total floating time: | 12 h |

### Comparative Example 2: Aggrenox^{®}200/25 mg (prolonged release profile)

### Dissolution test (0.1 N Hydrochloric Acid, 900 ml, basket-100 rpm):

| Time [hour] | % Drug Dissolved |
|---|---|
| 1 | 24 |
| 2 | 51 |
| 3 | 68 |
| 6 | 88 |
| 8 | 95 |

For the dissolution test, the intact capsule including dipyridamole containing pellets and acetylsalicylic acid containing tablets was measured.

## Claims

1. Intragastric floating tablet, comprising
a) in the intragranular phase: a pharmaceutically active ingredient and a water-swellable polymer, and
b) in the extragranular phase: hydroxypropyl methyl cellulose (HPMC),
wherein the tablet contains a gas generating agent.

2. Tablet according to claim 1, wherein the gas generating agent is exclusively contained in the extragranular phase.

3. Tablet according to claim 1 or 2, wherein the gas generating agent is selected from alkali metal or alkaline earth metal carbonates, e.g. sodium or calcium carbonate, alkali metal hydrogen carbonates, e.g. sodium bicarbonate, and alkali metal sulfites, e.g. sodium metabisulfite.

4. Tablet according to anyone of the preceding claims, wherein the intragranular phase further contains one or more fillers, preferably selected from microcrystalline cellulose, calcium hydrogen phosphate and lactose.

5. Tablet according to claim 4, wherein the mass ratio of the water-swellable polymer to the one or more fillers is in the range of 10 : 1 to 0.5 : 1, preferably 5 : 1 to 1 : 1, and more preferred 2 : 1 to 1 : 1.

6. Tablet according to anyone of the preceding claims, wherein the water-swellable polymer is selected from HPMC, polyethylene oxide (PEO), hydroxypropyl cellulose (HPC), methyl cellulose (MC), sodium carboxymethyl cellulose (CMC-Na), pregelatinized starch, tragacanth, pectin, polyacrylic acid, gelatine, xanthan gum, cross-linked polyvinylpyrrolidone (crospovidone), and mixtures thereof.

7. Tablet according to anyone of the preceding claims, wherein the tablet contains the pharmaceutically active ingredient in an amount of at least 40% by weight, preferably at least 50% by weight, and more preferred at least 55% by weight, based on the weight of the components constituting the intragranular phase.

8. Tablet according to anyone of the preceding claims, wherein the water-swellable polymer is HPMC.

9. Tablet according to anyone of the preceding claims, wherein the total floating time is from 5 h to 8 h, preferably from 5 h 30 min to 7 h 30 min, and more preferred form 6 h to 7 h, measured in 200 ml 0.1 N hydrochloric acid (pH 1.2) at 37± 0.5 °C.

10. Tablet according to anyone of the preceding claims, wherein 90 to 100% of the amount of the pharmaceutically active ingredient is released from the tablet after 5 h to 8 h, preferably 5 h 30 min to 7 h 30 min, and more preferred 6 h to 7 h, measured in 900 ml 0.1 N hydrochloric acid (pH 1.2) at 37 ± 0.5 °C (basket apparatus, 100 rpm).

11. Process for preparing a tablet according to anyone of the preceding claims, comprising the steps of:
i) preparing granules containing a pharmaceutically active ingredient, a water-swellable polymer and optionally a gas generating agent,
ii) providing a tablet mass containing the granules according to step (i), a gas generating agent and HPMC, and
iii) compressing the tablet mass.

12. Process according to claim 11, wherein the granules are prepared by wet granulation.

13. Process according to claim 12, wherein the wet granulation is conducted with an aqueous granulation liquid.

14. Process according to claim 13, wherein the granulation liquid is a mixture of water and an alcohol, preferably a mixture of water and isopropyl alcohol.

15. Pharmaceutical combination as a unit dosage form comprising (a) the tablet according to anyone of claims 1 to 10, wherein the pharmaceutically active ingredient is dipyridamole or a pharmaceutically acceptable salt thereof, and (b) an oral dosage form containing acetylsalicylic acid or a pharmaceutically acceptable salt thereof, said combination preferably being a capsule containing the tablet and the oral dosage form.
